# EUROPEAN PATENT APPLICATION

(11) **EP 1 361 280 A1**
(43) Date of publication of application: **12.11.2003**
(21) Application number: 02076803.2
(22) Date of filing: 08.05.2002
(51) Int. Cl.: C12P 23/00, B01D 11/04, C07C 403/00, C07C 403/24, C12S 3/02

(54) **Process for continuous production and extraction of carotenoids from natural sources**

(71) Applicant: Wageningen University, 6700 HB Wageningen (NL)
(72) Inventor: Wijffels, Rene Hubertus, 6711 NX Ede (NL); Hejazi, Mohammad Amin, 6702 BM Wageningen (NL); Holwerda, Evert Klaas, 6708 PS Wageningen (NL); Tramper, Johannes, 6703 EM Wageningen (NL)
(74) Representative: van Westenbrugge, Andries

(57) **Abstract**

The invention relates to a method for extracting carotenoids from a carotenoid producing viable cell The method comprises milking the cell in a culture medium consisting essentially of an aqueous phase and an biocompatible organic phase, followed by separating the organic phase from the aqueous phase.

## Description

### Field of the invention

The present invention relates to the extraction of carotenoids from natural sources. In particular it relates to the fermentative extraction of carotenoids from a natural source using an organic phase.

### Background of the invention

Carotenoids, naturally occurring pigments, are important nutritional and biological compounds. They are present in green and yellow vegetables and fruits such as carrots, citrus fruits, broccoli and spinach, but also some types of microalgae are very rich in carotenoids. These microalgae are autotrophic microorganisms which are able to use solar radiation as energy source and inorganic CO₂ as carbon source. Since both solar radiation and CO₂ are very abundant in nature and cheap, microalgae are a commercially and biotechnologically very interesting source of carotenoids. Nowadays, carotenoids, especially beta carotene, are extracted from mass cultivated halotolerant green algae from the genus *Dunaliella*.

US 4,680,314 describes a process which involves the direct oil extraction of carotene from harvested algae at 66°C. This process extracts not only the carotenes but also the chlorophyll in the algae.

In US 5,378,369 β-carotene is extracted from algal biomass using vegetable oil. In this method a vegetable oil is added to the algal biomass suspension to form a mixture of the organic phase and the aqueous suspension, whereby the β-carotene is caused to dissolve in the organic phase. The extraction is carried out at a temperature of up to 120°C, preferably 50-80°C in order to enhance beta-carotene solubility in the oil and to enhance lysis of the algal cells. This is followed by separation of the organic phase from the aqueous phase by microfiltration or ultrafiltration of the organic phase.

US 5,714, 658 describes a process for the extraction of carotenes from a fermentation residue by stirring this residue for several hours in a solvent mixture. The solvent is a mixture composed of acetic acid esters of C₁-C₄ alcohols and a portion of 1-25% by weight of oil of biological origin, for increasing the extraction capacity of solvent. The suitable temperature range for the extraction is between 50°C and 70°C.

US 5,310,554 describes a method for the preparation of highly purified natural beta-carotene and compositions containing the high purity natural beta-carotene. Beta-carotene is liberated from the cell by breaking the cells using hot water (60°C) and ultrafiltration. Carotenes are then extracted from the preparation by using a suitable organic solvent, such as hexane.

All publicly available methods for extraction of carotenoids from algal biomass comprise the destruction of the cells and in general several separation steps are necessary for the subsequent purification of the carotenoids. There are no methods available, which provide for the continuous production and extraction of carotenoids from natural sources by means of a simple, thus cost-efficient process.

### Description of the Figures

Figure 1: pigments content of the cells and organic biocompatible phase (HPLC analysis)
Figure 2: Beta carotene concentration in organic phase and aqueous phase (biomass)

### Detailed description

The invention relates to a method for extracting carotenoids from a carotenoid producing viable cell. The method comprises:
(1) milking the cell in a culture medium consisting essentially of an aqueous phase and a biocompatible organic phase , followed by
(2) separating the organic phase from the aqueous phase

In this context the 'milking' of the cells refers to the continuous production and extraction of carotenoids from growing or non-growing cells.

One advantages of the method according to the present invention is that it allows for long term and simultaneous production and extraction of carotenoids from living cells This is in contrast to existing methods, in which the cells are destroyed before or during extraction of the carotene.

Another advantage of the method according to the present invention is the integrated production and down stream processing which is an extreme simplification of the conventional multi-step extraction processes.

Yet another advantage of the method according to the invention is that it allows for the selective extraction of carotenoids over chlorophyll. In this way, conventionally used saponification steps after extraction have become superfluous.

Yet another advantage is that the method according to the invention allows for more efficient production of carotenoids, because it allows for the long-term production and extraction of carotenoids from suspended or immobilised cells

In this context 'viable cell' refers to a cell which is capable of producing beta-carotene. Classical natural carotenoid sources, such as algae may be used, but also other sources, which include but are not limited to yeast cells, fungal cells, bacterial cells, plant cells and cells from mammalian cell lines. These cells, including the algal cells, may be used as such or after being subjected to modification, such as e.g. modification by UV light or recombinant DNA technology.

Algae are preferably selected from the classes Chlorophyta or Rhodophyta, preferably they are algae from the genus Dunaliella, which includes but is not limited to the species Dunaliella (B), D. parva, D. tertiolecta, D. primolecta and D. salina. Also a mixture of these species may be used in the method of invention.

The carotenoids are preferably carotenes, more preferably beta carotenes. The method according to the invention may be used to extract one specific isomere, but also a mixture of isomeres.

Preferably a two-step method is applied: a first step which is a growth step to increase the number of cells and a second step for production and extraction of the carotenoid (the milking). These are just general indication, since growth is also possible during the production and extraction step.

In the first step, aqueous culture medium is used and in the second step a culture medium is used which essentially consists of an aqueous phase and a biocompatible organic phase. At the beginning of the second step, cells are preferably diluted with fresh medium. Suitable starting concentrations are from 1*10⁸ cells per liter.

Cells may grow autotrophically, heterotrophically or mixotrophically. The induction of the carotenoid production in the second step may be adapted to the way the cells are grown in the first step. For example, if cells are grown autotrophically, they are grown at low light intensity in the first step and at higher light intensity in the second step. The skilled person will know how to regulate the light intensity for the two steps on the basis of the cell concentration that he uses. The skilled person will also be familiar with other stress factors apart from high light intensity which will stimulate carotenoid production. These include but are not limited to nutrient deficiency and increased salt concentrations.

The two steps may take place in one compartment or bioreactor, but it is also possible to use more compartments or bioreactors. In a preferred embodiment, two bioreactors are used for the growth of the cell and production and extraction of the carotenoid. Since algae form a commercially very interesting source of carotenoids, the invention will be described for them, although it also works for other cells. The skilled person will know how to adapt the conditions and materials used for other cells once the details of the invention have been disclosed.

If algae are used, the aqueous phase of the culture medium may be any medium that is normally used for culturing algae. In a preferred example the aqueous phase of the culture medium contains 1M NaCl, 10 mM KNO₃, 1 mM NaH₂PO₄.2H₂O and 5 mM NaHCO₃ and trace elements, at pH 7.5. In the first step the algae are grown at low light intensity, preferably one starts at an average intensity of 0.5-3.0*10⁻⁸ micromol/cell.sec. In a second bioreactor, the cells are exposed to higher light intensity, preferably at an intensity of a least 4.5*10⁻⁸ micromol/cell.sec and a biocompatible organic phase is added. Higher light intensities may be used in the first step if it is desirable to stimulate carotenoid production already in the first step.

According to the method of the invention, the proportion of the biocompatible organic phase is preferably 10-60% by volume, more preferably 15-30% by volume or 15-20% by volume, most preferred is 20% by volume. The skilled person will understand that the less organic phase is used, the higher the volumetric concentration of carotenoids. However, at the same time, the more organic solvent used, the more carotenoids will be extracted.

Suitable organic phases are biocompatible, which means that they do not affect the viability of the cells. In the case of *Dunaliella* it will be an organic solvent with a log P_{octanol} of >6. This includes organic phases such as hexadecane and dodecane, which are the preferred ones. It also includes many vegetable oils, such as corn oil, soy oil, peanut oil, olive oil, sweet almond oil. And also microbial oils, from certain fungi, yeast and bacteria.

In general, organic phases with a log P_{octanol} value<6, such as hexane and acetone, are not suitable to be used in a *Dunaliella* culture medium, because they are not biocompatible and the cells will die.

The organic and aqueous phases are mixed during cultivation and extraction, preferably by recirculation because this has the concomitant advantage that the contact between the cells and the organic solvent is increased, which in turn increases the extraction rate.

In a preferred embodiment, the organic phase is replaced several times during the experiment after reaching a specific concentration to keep the extraction rate constant. The skilled person will understand that it is also possible to have a continuous flow of organic phase in and out.

The temperature of the culture medium during production and extraction of the carotenoids should never negatively affect the viability of the cells. In the case of *Dunaliella*, the temperature should not exceed 40°C, it is preferably kept between 24-30°C, most preferably it is maintained at 25-27°C so that the algae may keep on producing carotenoids, especially, beta carotene, during extraction.

According to the method of the invention, after extraction in the organic phase, the carotenoids may subsequently be extracted into a second organic phase. e.g. from dodecane into a vegetable oil, or be isolated.

After extraction in the organic phase from the culture medium, the carotenoids may subsequently be separated from this organic phase using both classic or advanced methods, which will be known to the skilled person.

These methods include but are not limited to evaporation under vacuum and inert gas, crystallisation, supercritical extraction of carotenoids and a combination of membrane separation and one of the former methods. When a membrane is used, usually the carotenoids will first be concentrated in a lipophilic membrane unit and then the retentate will be transferred to the evaporation or crystallisation units.

For more expensive products, e.g. production of different isomers with high purity, large-scale HPLC column may be applied.

If edible oil is applied as organic phase in the culture medium, separation of the carotenoids from the organic phase is not necessary, because an antioxidant (or vitamin) rich edible oil is directly produced by the method according to the invention.

### Examples

### Example 1 Organism, medium and culture conditions

*Dunaliella salina* (CCAP 19/18) was grown in a culture medium containing 1M NaCl, 10 mM KNO₃, 1 mM NaH₂PO₄.2H₂O and 5 mM NaHCO₃. 5 ml 1⁻¹ of trace elements stock with 12.3 mM Na₂EDTA.2H₂O, 4.66 mM FeCl₃.6H₂O, 42.0 mM CuSO₄.7H₂O, 60.6 mM ZnSO₄.7H₂O, 17.0 mM CoCl₂.6H₂O, 366 mM MnCl₂.4H₂O and 1.04 mM Na₂MoO₄ was also added. The pH of the medium after addition of 50 mmol of Tris-buffer was adjusted to 7.5 by some drops of a 3 M HCl. The medium was sterilised at 121°C for 40 minutes before inoculation. To avoid precipitation, the phosphorous was autoclaved separately and solid carbon sources (NaHCO₃) was put in the oven at 120 °C over night and then was mixed with the sterilised water.

Growth and beta-carotene production by *Dunaliella salina* were carried out in two different steps. Growth was carried out in the first step in a bubble-column bioreactor at low light intensity (450 micromol m⁻² s⁻¹). Then, the cells were transferred to the second bioreactor when the concentration reached 1.6 *10⁹ cell.l⁻¹. In the second bioreactor, which was a flat panel bioreactor with depth of 2.5 cm, the cells were diluted two times by fresh culture media. Beta-carotene production and extraction were performed in the second bioreactor at higher light intensity (1200 micromol m⁻² s⁻¹). Dodecane, which its biocompatibility for the cells of *Dunaliella salina* had been already approved, was applied as organic phase in the second step. The bioreactor contained 80% of aqueous phase and 20% of organic phase. Both mixing of the culture media and beta-carotene extraction, in the former step, were carried out by recirculation of the organic biocompatible solvent through the aqueous phase. The organic phase was replaced 3 times during the experiment when the concentration of carotenoids obtained was about 100 mg.l⁻¹. Cultivation of the algal cells in both steps was performed at 25-27°C.

### Example 2 Analysis of extracted compounds

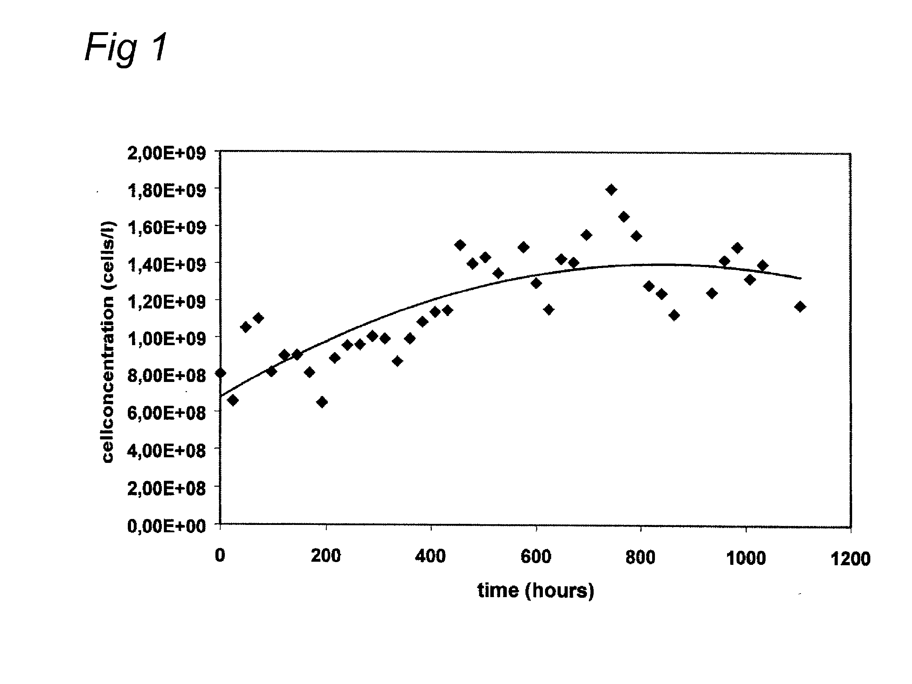

Both spectrophotometric and HPLC analysis showed that although the algal cells contain chlorophyll and carotenoids, only carotenoids can be extracted to the organic phase (Figure 1 for HPLC results). It means that there is a preference for the extraction of beta-carotene over chlorophyll by the biocompatible solvents, even though our estimations show that both chlorophyll and beta-carotene are very hydrophobe and have almost same log P_{octanol} values. The estimated log P_{octanol} values for chlorophyll and beta-carotene are respectively 17.2 and 17.6.

### Example 3 Effect of organic phase on algal cell growth

Cell growth was followed by regular sampling and direct counting of the cells under microscope during the 46 days of experiment. In the first week of experiment fluctuation in the cell concentration was observed. After that the cells showed very slow growth and in the last week of the experiment the cell population was almost constant. It is already known that the cell growth is limited when they produce carotenoids, but it seems that presence of organic phase has extra effect on the growth of the cells at higher light intensities.

### Example 4 Carotenoids production in the presence of organic phase

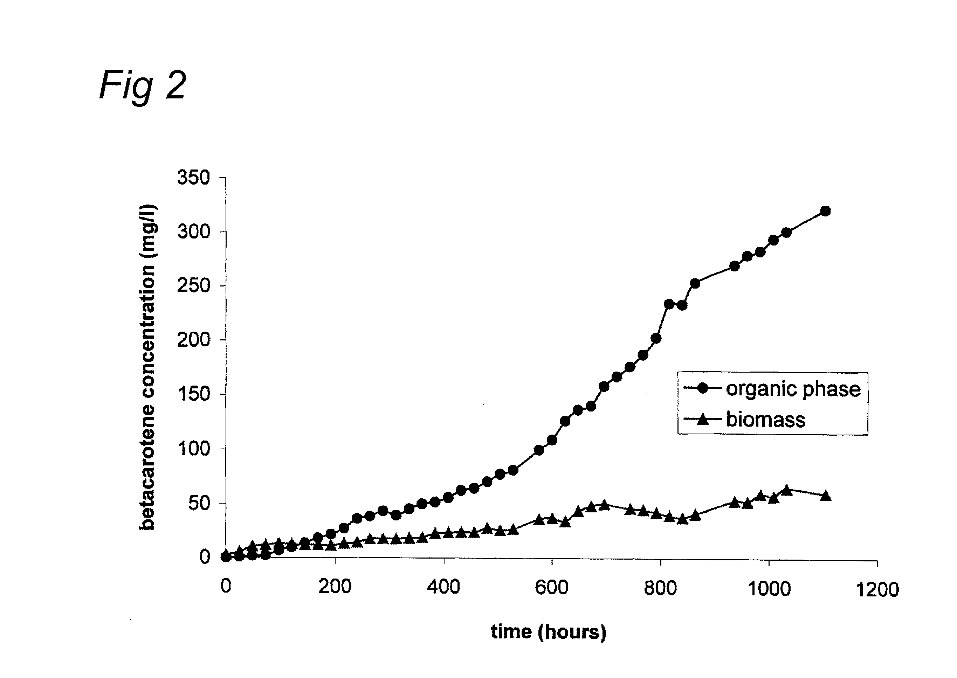

Figure 2 shows beta carotene concentration of the biomass and the organic phase as a function of time. It shows that the cells kept carotenoids production and accumulation for a long time in the presence of organic phase.

### Example 5 Carotenoid content of organic phase

Extraction efficiency was 56.5%. The extracted part contained high purity carotenoids which could be used directly after one simple separation operation. 33.5% of total carotenoids remained in the biomass which could be dried and utilised as carotenoid rich biomass.

## Claims

1. Method for extracting carotenoids from a carotenoid producing viable cell which method comprises:
- milking the cell in a culture medium consisting essentially of an aqueous phase and a biocompatible organic phase, followed by
- separating the organic phase from the aqueous phase

2. Method according to claim 1, wherein before or during the milking carotenoid production is extra stimulated.

3. Method according to claim 1 or 2, wherein the cell is an algal cell or a yeast .

4. Method according to claim 3 wherein the algal cell is an alga selected from the classes Chlorophyta or Rhodophyta.

5. Method according to claim 4, wherein the algal cell is of the genus Dunaliella, preferably D. salina.

6. Method according to claims 1-6 wherein the extracted carotenoid is a carotene, preferably a beta carotene.

7. Method according to claims 1-6 wherein the proportion of the organic phase is 10-60% by volume.

8. Method according to claim 1-7 wherein the organic phase is selected from the group: dodecane and hexadecane.

9. Method according to claim 1-7 wherein the organic phase is a vegetable oil.

10. Method according to claim 1-8 wherein the carotenoid is subsequently isolated from the organic phase or extracted from the organic phase with a vegetable oil.
